# EUROPEAN PATENT SPECIFICATION

(11) **EP 1 152 773 B1**
(45) Date of publication and mention of the grant of the patent: **05.01.2005**
(21) Application number: 00910149.4
(22) Date of filing: 11.02.2000
(51) Int. Cl.: A61L 2/08

(54) **METHODS OF INACTIVATING PATHOGENS USING BROAD-SPECTRUM PULSED LIGHT**
VERFAHREN ZUR INAKTIVIERUNG VON PATHOGENEN MITTELS BREITSPEKTRUM-PULSLICHT
INACTIVATION DE PATHOGENES PAR UTILISATION DE LUMIERE PULSEE A LARGE SPECTRE

(30) Priority: 13.02.1999 US 120034 P; 09.06.1999 US 329018
(43) Date of publication of application: 14.11.2001
(73) Proprietor: PUREPULSE TECHNOLOGIES, INC., San Diego, CA 92123 (US)
(72) Inventor: COVER, H., William, Escondido, CA 92026 (US); BOEGER, Jeffrey, M., Ramona, CA 92065 (US); MCDONALD, Catriona, J., San Diego, CA 92122 (US); MALOY, Sean, San Diego, CA 92131 (US); BUSHNELL, Andrew, Hugh, San Diego, CA 92131 (US); COOPER, James, Randall, San Diego, CA 92103 (US)
(74) Representative: HOFFMANN - EITLE
(86) International application number: PCT/US2000/003546
(87) International publication number: WO 2000/047240

(56) References cited:
- WO-A-96/00091
- DE-A- 3 505 728
- US-A- 5 034 235

## Description

### BACKGROUND OF THE INVENTION

The use of biologically derived compositions in scientific research and in the manufacture of pharmaceutical/therapeutic substances is ubiquitous. Human blood is routinely used as a source of protein agents for treatment of various diseases and disorders; for example, blood plasma is fractionated to provide Factor VIII, Factor IX, antithrombin, transferrin, albumin, immunoglobulins and platelets, as well as other therapeutic agents. Tissue culture methods are commonly employed in the production of numerous pharmaceutical/therapeutic agents and related compositions, such as recombinant DNA and/or recombinant protein from genetically engineered cell lines, virus vectors, amino acids, peptones, insulin and monoclonal antibodies. Similarly, animal-derived reagents, such as bovine serum albumin (BSA) and sheep blood components are routinely used in research and manufacturing.

Because these compositions are derived from living organisms, including humans, animals and plants, they can be contaminated with one or more viruses, bacteria or other pathogens. Should a human or other animal come into contact such a contaminated product, infection, disease and even death of the individual can result. This risk of infection is of particular concern where the individual must be routinely treated with a composition for a prolonged period of time; for example, some hemophiliacs are regularly treated with blood-derived compositions for their entire lives. similarly, persons having severely compromised immune systems, such as those with Acquired Immune Deficiency Syndrome (AIDS), are at particular risk for infection from exposure to contaminating pathogens in biologically derived compositions. Animals being treated with veterinary pharmaceuticals are likewise at risk from contaminated compositions. In addition to posing a risk of infection to the individual receiving the biologically derived composition, the effectiveness of the composition itself may be compromised by the presence of viral, bacterial and other pathogenic contaminants.

Biologically derived compositions are not only used in the production of pharmaceutical/therapeutic substances, but such products are also frequently used as reagents (or to produce reagents) for biological research. When a biologically derived reagent is contaminated with a virus, bacteria or other pathogen, the research project for which it is employed can be rendered useless, inconclusive or, even worse, misleading. Blood products that are frequently used in manufacturing and research include BSA and human blood plasma proteins.

Recombinant DNA and proteins, monoclonal antibodies, viral vectors and related compositions are also examples of biologically derived compositions used in both research and manufacturing processes. While these products are less likely to be contaminated by viruses, bacteria and/or other pathogens, such contamination is none the less possible. Additionally, when such compositions are used as tools in research, if they become contaminated, they may pose a significant risk to such research, making contamination of concern here.

Therefore, biologically derived compositions, particularly those of human or animal origin, including for example cell lines, blood and/or plasma, require stringent viral safety assessments prior to marketing. Current approaches to providing such safety assurance include testing source materials and product for viruses, bacteria and/or other pathogens at specific manufacturing steps, using infectivity assays or other diagnostic tests. The effectiveness of testing materials is limited by available test methodology, assay sensitivity and the possibility of an undetectable unknown or mutated pathogen, especially a virus. Therefore, in developing manufacturing processes, process steps are included that are designed to remove or inactivate viruses and/or other pathogens from such biologically derived composition. These processes are tested for their capacity to remove or inactivate viruses to the level of safety required or most appropriate. The sum of the logarithmic reductions for each process step determines the overall effectiveness of viral clearance. For example, at least a one log reduction in viral content is required for each process step.

Currently, numerous methods of decontaminating or sterilizing biologically derived compositions are available. Generally, these treatment methods include physical methods, chemical methods, heat methods, irradiation methods and, preferably, some combination thereof. Prolonged heating of biologically derived compositions; treatment of such compositions with one or more chemical agents combined with heating or irradiation thereof; and use of solvent-detergent methods of viral inactivation are three of the most commonly used methods of decontamination of biologically derived compositions. However, no single method of deactivation of pathogens, especially viruses, in biologically derived compositions has been proven successful against a broad-spectrum of subsequently pathogenic agents. Furthermore, most of these methods require further processing of the biological composition in order to remove the chemicals employed in deactivating the viruses, bacteria and/or other pathogens. Frequently, these additional processing steps include one or more extraction steps and/or chromatography steps, which dramatically increases the cost of manufacturing the final product and, therefore, increases the retail price of the product to the customer.

Exemplary physical methods available to remove or inactivate viruses and/or other contaminants include highly specialized filtration methods, affinity chromatography, ion exchange chromatography and polyethylene glycol fractionation. However, there are many limitations to these physical methods, particularly when many compositions cannot be so filtered. Thus, to the degree filtration, chromatography and other physical methods are used, they are frequently one in a series of decontamination procedures. For example, to produce relatively pure solutions of Factor VIII, Factor IX or immunoglobulin, blood plasma may be first subjected to a solvent-detergent treatment protocol, then chromatographically treated and finally further filtered to yield the desired final product. Ultrafiltration may be used to eliminate parvovirus from blood or blood products, but this method is only partially effective. For example, it can be applied to Factor IX concentrates, but not to Factor VIII.

Other well known methods of removing or inactivating viruses, bacteria and/or other pathogens contaminating biologically derived compositions are described, for example, in U. S. Patent No. 4,540,573 (Neurath, *et al*.), U.S. Patent No. 4,946,648 (Dichtelmüller, *et al*.), U.S. Patent No. 5,418,130 (Platz, *et al*.), U.S. Patent No. 5,527,704 (Wolf, Jr., *et al*.), U.S. Patent No. 5,663,043 (Zepp, *et al*.) and U.S. Patent 5,866,316 (Kempf, *et al*.). These patents describe combination treatments of biologically derived compositions, particularly mammalian blood products, such as plasma, plasma fractions or blood cellular matter (erythrocytes, platelets or protein fractions), to inactivate viral and/or bacterial contaminants therein. Further, all of the described methods include the use of at least one chemical agent to either directly degrade the contaminating organism or to sensitize it for degradation by another chemical agent or heat or radiation.

Use of such combined treatment methods are more effective and more reliable with respect to some viruses than treatment with a single chemical agent. For example, experiments have shown that cold treatment with β-propiolactone alone provided only 0-3 log inactivation of HIV and SV40 in plasma (Scheidler et al., 1998), and that treatment of blood plasma with ultraviolet radiation alone provided protection from hepatitis infection to only about 40% of the recipients; whereas, an infection rate of zero resulted from use of plasma treated with both β-propionolactone and ultraviolet radiation. (Lo Grippo, et al., "Human Plasma Treated With Ultraviolet and Propiolactone - Six year Clinical Evaluation." JAMA 1987:722-726 (1964).) Thus, understandably, use of a single chemical agent alone to inactivate viral contaminants in biologically derived compositions is becoming less preferred.

U.S. Patent Nos. 4,726,949, 4,866,282 and 4952,812, Miripol, *et al*. (hereinafter, collectively, the Miripol patents) describe the irradiation of a thin layer of white blood cells with ultraviolet radiation predominately of a wavelength of 280 nm to 320 nm, for about 0.25 to 15 minutes, in order to cause the white blood cells to substantially lose their capability to set off an immune reaction in an alloimmunized patient. The Miripol patents teach that ultraviolet bulbs emitting significant energy at a wavelength of 254 nm are to be avoided in practicing the described methods as light at such a wavelength causes damage to blood cells. Similarly, the UV-A range (about 365 nm) is identified undesirable as it does not provide good reduction of the lymphocyte alloimmunization effect. Although the Miripol patents describe a method of irradiating a blood product (namely white blood cells) with ultraviolet radiation, there is no indication that such methods would be useful for decontamination of biologically derived compositions by inactivating viruses, bacteria and/or other pathogens, nor is there any indication that broad-spectrum pulsed light, which necessarily includes light in both the 254 nm and 365 nm ranges, could provide improved decontamination as compared to ultraviolet radiation alone.

WO-A-9600091 further describes a method of virus inactivation in samples having biologically active components derived from potentially virus infected sources by differentially irradiating the sample or sources with electromagnetic radiation generated by a tunable laser-device for inactivating the virus but not the activity of the biologically active components. The laser radiation has a wavelength in the range of 300 nm to 370 nm.

While various methods of decontamination of biologically derived compositions are known and used, no method has been developed that is reliably effective against most pathogenic microorganisms and that may be safely used on biologically derived compositions including therapeutic proteins and/or other biomolecules. Heat treatments generally require a great deal of time and can be detrimental to the protein or other agent sought to be recovered; for example, plasma protein fractions and human albumin require heating at 60°C for 10 to 11 hours to inactivate viruses. Parvovirus, hepatitis A and other non-lipid enveloped viruses have been found to be resistant to solvent/detergent treatments, and parvovirus has also been demonstrated to also be resistant to heat inactivation.

Given the prevalance of decontamination methods by adding chemical agents to the biologically derived composition, a number of methods have been developed for removing or deactivating these compositions after such decontamination treatment. For example, U.S. Patents Nos. 4,540,573 (Neurath, et al.), 4,789,545 (Woods, et al.) and 5,817,765 (Isaksson, et al.) describe different methods of separating a biologically derived final product from the chemical contaminants present therein as a result of treatments to deactivate pathogenic contaminants. Obviously, the need to perform additional processing steps in order to remove previously added chemical agents from a biologically derived composition is undesirable.

While bacteria and other pathogenic contaminants are of concern in manufacturing and using biologically derived compositions, viruses are often of greatest concern. Viruses are frequently grouped based upon their genome, *i.e*., DNA or RNA viruses, and/or according to the physical characteristic of being enveloped or non-enveloped. Further, individual viruses are generally categorized into a family of viruses with which they share certain evolutionary characteristics. Thus, for example, viruses in the herpes virus family (Herpesviridae) are enveloped DNA viruses and viruses in the Adenoviridae family are non-enveloped DNA viruses. Examples of enveloped and non-enveloped RNA virus families are, respectively, the Flaviviridae family (which includes Yellow Fever virus, Hepatitis C virus and Bovine Diarrhea virus) and the Picornaviridae family (which includes Poliovirus, Rhinovirus and Hepatitis A virus). Naturally, those viruses that are most virulent to humans and/or most prevalent in biologically derived compositions are of greatest concern in developing and implementing decontamination procedures. Such viruses include, for example, Parvovirus, Simian Vacuolating Virus (SV40), Human Immunodeficiency Virus (HIV), Hepatitis Viruses and Bovine Viral Diarrhea Virus (BVDV).

Parvovirus is a non-enveloped DNA virus. Infection with human-B19 parvovirus may result in miscarriage, or in persistent anemia in those infected with HIV. Parvovirus may be transmitted by blood or blood products, and is resistant to both heat treatment and solvent/detergent treatment. It has even been transmitted with compositions treated by a combination of two inactivation methods. Canine parvovirus (CPV) is ubiquitous in the environment and is a serious veterinary pathogen against which vaccines are required. Significantly, photoinactivation methods which have been demonstrated capable of inactivating HIV were not effective against CPV (Hirayama et al., 1997).

Simian Vacuolating Virus (SV40) is a non-enveloped DNA virus. Inadvertent human exposure to SV40 occurred in the late 1950s and early 1960s when polio and adenovirus vaccines prepared in rhesus monkey cells containing SV40 were used, Shah, K., et al., Am. J. Epidemiology, 103:1-12 (1976). While epidemiologic surveys have found no discernible relationship between exposure to SV40 and development of tumors (in particular, no association between exposure to SV40-contaminated polio vaccines and the incidence of tumors of the brain and ovaries was found), recently, DNA sequences related to SV40 have been detected in a variety of human tissues. For example, SV40-related DNA has been found in choroid plexus tumors, ependymomas, mesotheliomas and osteosarcomas. See, for example, Bersagel, et al., NEJ Med., 326:988-93 (1992); Carbone, et al., Oncogene 9:1781-90 (1994); Cristando, et al., J. Environ. Pathol Toxicol oncol., 14:29-34 (1995); and Martini, et al., Cancer Res., 56:4820-25 (1996). Further infectious SV40 has been isolated from a choroid plexus tumor, Lednecky, et al., Virology, 212:710-717 (1995). It is unclear under what circumstances and to what extent SV40 contaminants in biologically derived compositions can infect recipients of that product. Thus, it is advantageous to make an effort at removing and/or inactivating SV40 present in biologically derived compositions prior to administration of the same. Unfortunately, SV40 has been found to be relatively heat resistant and to be inconsistently inactivated by β-propiolactone treatment. See Lelie, et al., J. Med. Virol., 23(3):297301 (Nov. 1987) and Sheilder, et al. Biologicals, 26(2):135-144 (June 1998)

Human Immunodeficiency Virus (HIV) is a well known non-enveloped RNA virus that causes disease and death in humans. Under appropriate conditions, HIV can be inactivated by gamma irradiation (Salai et al., Ann. Transplant, 2(1):55-56, 1997), and by some chemicals (Dewilde et al., Biol. Chem., 379(1):1377-79, 1998, Arthur et al., AIDS Res. Human Retroviruses, 14 (Suppl. 13):5311-19 (Oct. 1998). However, HIV-2 shows only limited inactivation with beta-propiolactone (Scheidler et al., Biologicals, 26(2):135-44 (June 1998). Similarly, heat treatment of HIV has been shown to be effective in only some compositions (Azari et al., Artif. Cells Blood Substit. Immobil Biotech, 26(5-6):577-82 (Nov. 1998); whereas both HIV-1 and -2 can be inactivated by an appropriate solvent/detergent within a few minutes (Biesert, et al. Vox Sang 74 (Suppl 1):207-212 (1998). It has also been reported that HIV can be inactivated by a combination treatment of visible light and the dye, methylene blue (Muller-Breitkreutz and Mohr, 1998).

Bovine Viral Diarrhea Virus (BVDV) is an enveloped RNA virus from the Flavivirus family that causes severe disease in cattle. Because this virus is able to cross the placenta and infect the bovine fetus, it is estimated that between 10% and 75% of commercial products of fetal bovine serum are contaminated with BVDV. While it is unclear whether, under what circumstances and to what degree BVDV can infect humans, its presence has been linked to infantile gastroenteritis and microcephaly in humans (Harasawa, 1997). Of additional concern is the fact that non-cytopathic strains of BVDV can incorporate host cell RNA into their genomes, thereby raising the issue of oncogenicity within continuous cell lines contaminated with this virus. Limited inactivation of BVDV has been achieved using chemicals, such as β-propiolactone (Scheidler et al., Biologicals, 26(2):135-144 (June 1998). Additionally, heat treatment at 74°C for 90 minutes has been shown to accomplish a 7 log reduction of BVDV (Azari et al, Actif. Cells Blood Substit. Immobil. Biotech. 26(5-6):577-82 (Nov. 1998). Obviously, however, such severe and prolonged heat treatment is not appropriate for many contaminated compositions.

Broad-spectrum pulsed light (BPL) provides an approach for deactivation of microorganisms using high-intensity, short-duration pulses of incoherent, polychromatic light in a broad-spectrum. BPL is different from continuous, non-pulsed UV light in a number of ways. The spectrum of BPL contains UV light, but also includes a broader light spectrum, in particular between about 180 nm and about 2600 nm. The spectrum of BPL is similar to that of sunlight at sea level, although it is 90,000 times more intense, and includes UV wavelengths between 200 and 300 nm which are normally filtered by the earth's atmosphere. BPL is applied in short duration pulses of relatively high power, compared to the longer exposure times and lower power of non-pulsed UV light. See for example U.S. Patent Nos. 5,034,235 (Dunn et al.), 5,489,442 (Dunn et al.), 5,768,853 (Bushnell et al.) and 5,786,598 (Clark et al.).

BPL has been used to decontaminate and/or sterilize various target objects, such as food products, packages, water and other fluid, semifluid and solid objects. Primarily, such is accomplished by placing the target object into or passing target object through a BPL sterilization chamber, and exposing the object to an appropriate number of flashes of BPL at an appropriate energy level. See, for example, the above four patents and 5,900,211 (Dunn, et al.). Importantly, however, the use of BPL to decontaminate biologically derived compositions has not heretofore been described or contemplated. While BPL has been demonstrated to be useful in deactivating various microorganisms on the surface of solid objects and/or within certain relatively transparent fluids, there has been no suggestion that BPL might be useful in decontaminating biologically derived compositions containing proteins and/or other biomolecules of interest. Because biologically derived therapeutic and pharmaceutical compositions are often administered intravenously and, in any case, in a concentrated form, complete removal of contaminating microorganisms is critical to safety assurance. Similarly, the process of decontamination must be selected to effectively and reliably decontaminate the biologically derived composition without adversely affecting the effectivity of the final product.

BPL provides biological effects which are different from non-pulsed UV light. For example, pigmented bacteria, such as *Aspergillus niger*, are known to be more resistant to UV radiation than are bacillus spores. In studies using BPL, however, *Aspergillus niger* was more sensitive, on dry surfaces, to BPL than were three different bacillus spores: *Bacillus stearothermophilus, Bacillus subtilis* and *Bacillus pumilus*. Further, conventional UV treatment injures DNA by mechanisms that may be reversed under certain experimental conditions classified as either "dark enzymatic repair" or "light enzymatic repair" (Block, S., Disinfection, Sterilization and Preservation, 4^{th} ed., Williams and Wilkins, U.S.A. (1991)). This photoreactivation by either dark or light enzymes does not occur when BPL is used to treat the same microorganism (such as, *Bacillus subtilis, Bacillus pumilus, Aspergillus niger, Clostridium sporogenes*, *Candida albicans, Staphylococcus aureus, Escherichia coli, Salmonella choleraesuis* and *Pseudomonas aeruginosa*). (Furukawa, et al., "Brand New Pulsed Light Sterilization Technology Can Sterilize Both Injectable Solution and its 2 mL Polyethylene Container", presented at the PDA International Congress, Japan (Feb., 1999)). The presence of wavelengths in the visible range further differentiates BPL from UV light as does the means for generating the two different lights. UV light is usually generated using mercury lamps, which pose some safety hazards; whereas BPL is generated by lamps using an inert gas, i.e. xenon.

Thus, what is needed is a new method of inactivating viruses, bacteria and/or other pathogens contained in biologically derived compositions, which is effective against a variety of pathogens, especially a variety of viruses, and which may be employed on a variety of compositions without unnecessarily compromising the effectiveness of the final product.

### SUMMARY OF THE INVENTION

The present invention addresses the above and other needs by providing a method for the decontamination of biologically derived compositions using high-intensity, short-duration pulses of incoherent, polychromatic light in a broad-spectrum.

According to a first aspect, the present invention provides a method for reducing the content of a pathogen present in a biologically derivable composition comprising illuminating the composition with at least one high-intensity, short duration pulse of incoherent polychromatic light in a broad spectrum wherein the light intensity is at least 0.01 J/cm², the pulse duration is less than 100 ms and the light wavelengths are between 170 nm to 2600 nm, such that the content of pathogen is reduced by a factor of at least 10,
wherein the biologically derived composition is a blood plasma composition, a Factor VIII composition, a Factor IX composition, an antithrombin, an immunoglobulin composition, a monoclonal antibody composition, a viral vector composition, a heparin composition, a collagen composition, an insulin composition or a blood serum albumin composition.

Preferably in this method, the pathogen is a virus, bacteria, pyrogen, toxin, fungi or a prion. In case the pathogen is virus, then it is preferably selected from human immunodeficiency virus (HIV)-1, HIV-2, hepatitis A, hepatitis B, hepatitis C, HTLV-I, HTLV-II, cytomegalovirus, simian vacuolating virus 40, bovine viral diarrhea virus and canine parvovirus.

It is preferred that the illuminating step comprises illuminating the biologically derived composition with at least two high-intensity, short duration pulses of incoherent polychromatic light in a broad spectrum.

It is further preferred that the biologically derivable composition comprises a biomolecule of interest which is not destroyed by the illuminating step.

According to a further preferred aspect of the invention, the method further comprises the steps of:
providing a broad-spectrum pulsed light apparatus comprising a treatment zone including at least one flashlamp and two opposing transmissive plates; and
causing the biologically derived composition to flow between the two opposing transmissive plates; wherein said illuminating of said biologically derived composition is performed while it is located between the two opposing transmissive plates.

Preferably the biologically derivable composition is bovine serum, sheep blood, bovine insulin, bovine transferrin, bovine heparin, collagen, amino acids, peptones, peptides or monoclonal antibodies or proteins from a genetically engineered mammalian cell line. In this aspect, it is further preferred that the pathogen is a virus and the biologically derived composition is bovine serum, sheep blood bovine insulin, bovine transferrin, bovine heparin, collagen, amino acids, peptones, peptides or monoclonal antibodies.

The method of the present invention preferably results in at least one log reduction, more preferably at least two log reductions, in the active pathogen content of the initial biologically derivable composition.

In particular, proteins, polysaccharides, nucleic acid lipids, antibodies and other biomolecules of interest present in the biologically derived composition do not lose properties essential for their intended use, that is the biomolecules of interest are not irreversibly altered, for example, so that they would no longer exhibit their desirable bioactivity, by exposure to such broad-spectrum pulsed light. Thus, advantageously, the biologically derived composition requires few, if any, additional processing steps as a result of such broad-spectrum pulsed light treatment.

Therefore, provided herein are fast, efficient and reliable methods for inactivating viruses, such as, for example, HIV-1 or -2; hepatitis A, B or c, HTLV-I or II or cytomegalovirus, present in biologically derived products, such as blood plasma, by exposing the product to at least one high-intensity, short-duration pulse of BPL, i.e. incoherent, polychromatic light in a broad-spectrum. In a further aspect, provided herein are similarly fast, efficient and reliable methods for the inactivation of viruses, such as, for example SV40 and/or related blood-borne viruses present in an animal blood-derived composition by exposing the composition to BPL.

According to a preferred aspect, the method of the invention further comprises the steps of:
diluting the biologically derived composition prior to said illuminating step; and
concentrating the biologically derived composition after said illuminating step.

In this aspect, the present invention is useful for decontaminating biologically derived compositions that are somewhat non-transmissive to broad-spectrum light and thus might not be sufficiently decontaminated if not first diluted. Additionally, where the protein content of the biologically derived composition is particularly high, for example greater than about 10%, it may be desirable to first dilute the composition prior to illuminating the same with BPL in order to both better control the effects of the BPL on the proteins and to better control the inactivation of the pathogens present in the composition.

Further provided herein are methods for the inactivation of a plurality of contaminating microorganisms present in biologically derived compositions, wherein the microorganisms include at least two selected from the group consisting of viruses, bacteria, fungi and prions and wherein proteins, peptides and/or other biomolecules of interest in the composition are not irreversibly altered, and thus remain effective for their intended purpose, the method comprising exposing the biologically derived composition to at least one pulse of broad-spectrum light.

In a still further aspect, provided herein are methods of inactivating at least one microorganism present in a biologically derived composition by exposing the composition to high-intensity (*i*.*e*., 0.01 J/cm² to 50 J/cm², *e*.*g*., 0.05 J/cm² to 1.0 J/cm², wherein energy density is measured at the surface of the target object), short duration (*i*.*e*., 10 ns to 100 ms, *e*.*g*., 0.3 ms), pulses of incoherent, polychromatic light in a broad-spectrum (*i*.*e*., 170 nm to 2600 nm; 1.8x10¹⁵ Hz to 1.2x10¹⁴ Hz). Generally, 1 to 5 pulses will be used.

### BRIEF DESCRIPTION OF THE DRAWINGS

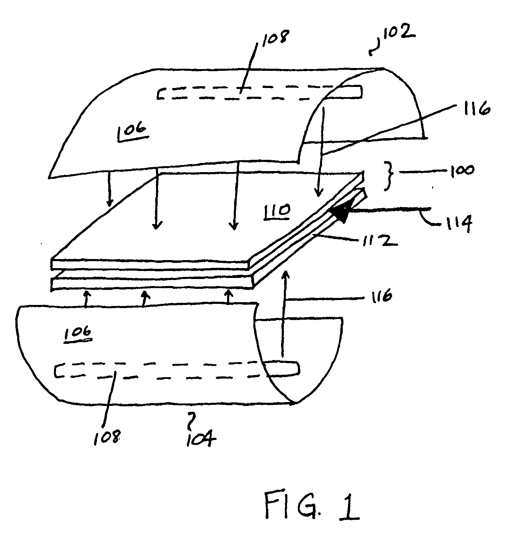

FIG. 1 is a schematic view of the treatment zone of an exemplary pulsed light processing apparatus that may be used to decontaminate biologically derived compositions in accordance with the present invention, which apparatus employs thin, closely spaced plates within which the composition resides as it is treated;

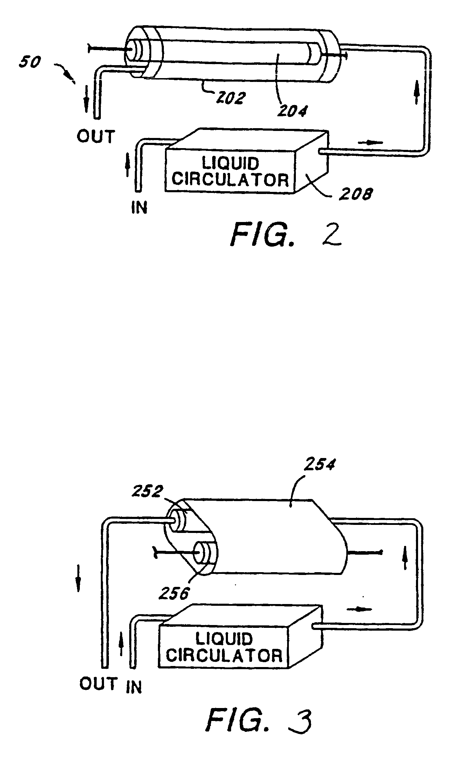

FIG. 2 is a schematic view of another exemplary pulsed light processing apparatus useful herein, which apparatus treats biologically derived compositions flowing longitudinally through a jacket surrounding an elongated, incoherent pulsed light source that provides high-intensity, short-duration pulses of polychromatic light in the broad-spectrum;
FIG. 3 is a schematic view of still another exemplary pulsed light processing apparatus, wherein biologically derived composition flows in a direction parallel to one or more elongated incoherent light sources, within an elliptical reflector, and is treated with high-intensity, short-duration pulses of polychromatic light in a broad-spectrum; and

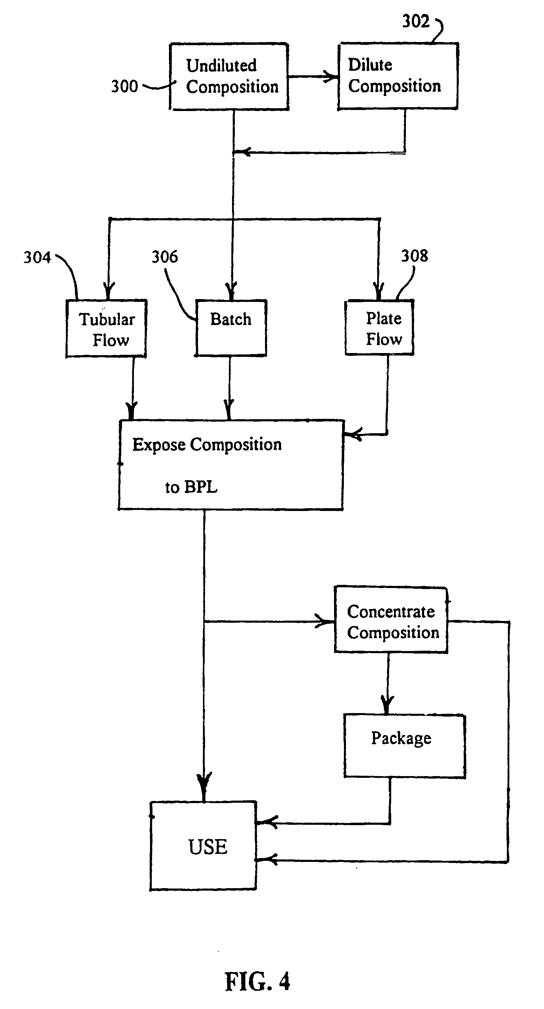

FIG. 4 is a flow chart illustrating some of the various steps that may be utilized in preparing a biologically derived composition for decontamination with pulsed light, exposing such composition to the pulsed light and thereafter processing the decontaminated composition.

### DETAILED DESCRIPTION OF THE PREFERRED EMBODIMENTS

The present invention provides a method for quick, reliable and efficient inactivation of pathogens present in biologically derived compositions. It has been found that high-intensity, short duration pulses of incoherent polychromatic light in a broad spectrum can be used to inactivate viruses, bacteria and other pathogens (which for purposes of this application should be understood to include pyrogens, toxins, fungi and prions) present in a biologically derived composition, such as, for example, blood-derived compositions, compositions derived from genetically engineered cell lines, compositions derived through tissue culture processes and related products, generally without destroying desirable biological properties of biomolecules of interest, such as for example, proteins, polysaccharides, antibodies, nucleic acid lipids, amino acids and/or peptones in the composition.

In particular, a biologically derived composition is placed into and/or flowed through a broad-spectrum pulsed light treatment apparatus. Within a treatment zone of the apparatus, the composition is illuminated by at least one, preferably two and most preferably three short duration (e.g., less than about 100 ms, preferably about 0.3 ms) pulses of high-intensity (e.g., 0.01 J/cm² to 50 J/cm², e.g., 0.05 J/cm² to 1.0 J/cm², measured at the surface of the composition) incoherent polychromatic light in a broad spectrum (e.g., 170 nm to 2600 nm; i.e., 1.8 x10¹⁵ Hz to 1.2 x 10¹⁴ Hz). Overall, it is anticipated that commercial methods may employ 1-5 short pulses. As a result of such illumination, the content of active pathogen within the biologically derived composition is reduced by at least one log (i.e., a factor of 10) and preferably is reduced by more than 2 logs (i.e., a factor of 100). Most advantageously, this treatment method can be easily and quickly administered at various points during the processing of the biologically derived composition, thereby providing further assurance of complete or near complete inactivation of pathogens in the composition.

Various apparatus may be employed to practice these pathogen reducing methods. Apparatus designed to provide high-intensity, short duration pulsed incoherent polychromatic light in a broad-spectrum are described, for example, in the '235 patent, '442 patent, '853 patent, '442 patent, '853 patent, '598 patent and Patent No. 5,900,211. Common to the apparatus used to provide broad-spectrum pulsed light treatment are that the treatment chamber is light-tight; flashlamp(s) are positioned within the apparatus such that the light emitting therefrom is optimally directed at the target object; reflective material is preferably employed to further maximize the pulsed light directed towards the target object; and transmissive materials (*i.e.*, quartz, sapphire or similar material) are employed where material is required between the flashlamps and target object (such as supporting structures for the target object within the chamber), so that interference with the BPL is minimized.

Of particular importance in designing or selecting apparatus for BPL treatment is the configuration of the treatment zone of the apparatus, that is, the area within the apparatus where the target object is to be illuminated with the pulsed light. Because the pulsed light must illuminate all surfaces of the target object, the treatment zone will generally be designed to maximize the pulsed light directed to the target object. For example, where more than one flashlamp is employed simultaneously to decontaminate the target object, the treatment zone will preferably be designed such that the target object is about equidistant from each flashlamp and flashlamps are preferably positioned to surround the target object. In order to ensure that pulsed light illuminates the entirety of the target object, structures employed to support the target object within the treatment zone are preferably formed of material that is at least about 1%, preferably at least about 10%, more preferably at least about 50% and most preferably at least about 85% transmissive to BPL. The following descriptions are of exemplary apparatus that may be employed in the sterilization or decontamination of biologically derived compositions in accordance with the present invention.

FIG. 1 shows a schematic view of a treatment zone of a preferred exemplary apparatus for use in treating biologically derived compositions with BPL. In this preferred apparatus, two thin (i.e., less than about 5 mm, for example, about 2 mm) transmissive plates 110, 112 are positioned generally parallel to one another and spaced very closely together (i.e., less than about 5 mm apart, e.g. about 1 mm) to provide a passageway or conduit 100 through which the composition to be treated may pass. on either side of the plates 110, 112 are two flashlamp systems 102,104, each including a reflector 106 partially surrounding a flashlamp 108. Such flashlamp systems are commercially available, for example, from PurePulse Technologies of San Diego, California as PUREBRIGHT Model No. PBS-1.

The flashlamps 108 are preferably elongated, and the flow of the biologically derived composition (illustrated by the arrow 114 in FIG. 1) is preferably parallel to the elongated flashlamps. The plates 110, 112 are preferably fixed to each other along the two opposing edges that are parallel to the flashlamp so that a composition passing therebetween is contained therein. A suitably transmissive gel, such as for example, agarose, may be employed. In lieu of parallel plates, a generally rectangular transmissive conduit (*e.g.*, quartz or sapphire) may be employed to transport the composition through the illustrated treatment zone. While only two flashlamp systems 102, 104 are illustrated, additional systems may be employed; for example, the conduit 100 may be surrounded by pulsed light sources. In either case, the light emitted from the flashlamp systems 116 is directed towards the biologically derived composition as it passes through (or resides in) the conduit 100, thereby inactivating viruses and/or other microorganisms contained therein. Advantageously, by spacing the plates 110, 112 of the conduit 100 close together, the composition being treated is spread into a thin layer, thereby facilitating exposure of the entirety of the composition to the pulsed light, even when the composition is relatively non-transparent and/or undiluted.

FIG. 2 shows a schematic view of another exemplary apparatus 50 that may be employed to inactivate viruses and/or other contaminating microorganisms in the composition. The apparatus 50 comprises a reflective, cylindrical enclosure defining a treatment chamber 202 through which the composition flows surrounding a pulsed light source 204. The pulsed light source is a high powered xenon flashlamp provided with a suitable power source (not shown) in accordance with conventional practice for flashlamp operation.

A liquid circulation pump 208 controls the flow rate of the composition through the treatment chamber 202 in relation to the pulse repetition rate of the pulsed light source 204 so that, during the time the composition resides within the treatment chamber 202, all of the product that passes therethrough is exposed to a predetermined number of high-intensity, short-duration pulses of incoherent polychromatic light in a broad-spectrum. Very generally, the composition being treated will be pumped through the chamber at a flow rate of about 1-4 liters/min. The composition exiting the treatment chamber 202 is therefore decontaminated and generally non-infectious.

The product treatment chamber 202 may be arranged so as to be separate from the pulsed light source 204 to prevent the composition therein from contacting the light source 204. Such may be achieved, for example, by employing a quartz jacket (or quartz cylinder) around the light source 204, with the composition being treated passing outside the quartz jacket. Cooling water may be circulated between the light source 204 and the quartz jacket.

The diameter of the treatment chamber will vary depending upon many factors including but not limited to the specific absorption characteristics of the composition to be treated, the physical and operating characteristics of the light source 204, i.e., flashlamps, and the degree of product mixing between pulses, i.e., flashes, of light. The treatment chamber 202 may include a reflector assembly as its outer wall or as an external reflector, in order to reflect illumination traversing the composition back toward the composition flow path. When an external reflector is used, the reflector assembly may include a quartz (or other transmissive material) cylinder (or tube) inside which the composition is circulated and outside of which the external reflector is positioned.

It is noted that some biologically derived fluids, either natural or following dilution, are relatively transparent to light, including significant portions of the UV spectrum. Accordingly, there is relatively little attenuation through absorption in such compositions, with the flux density decreasing largely only as a function of distance from the light source. However, for other compositions that have significant absorption, flux density will decrease as a function both of distance from the flashlamp and of such composition's absorption. In any event, a desired minimum flux density, e.g., 0.4 or 0.5 J/cm² (or even as low as 0.1 or 0.2 J/cm² depending on the particular microorganisms to be deactivated) should be maintained throughout the treatment zone. Alternatively or in addition, mixing should occur to insure that all of the fluid being treated is subjected to an appropriate flux intensity and number of pulses to achieve the desired degree or level of inactivation, i.e., kill or sterilization.

While the flashlamp 204 is located internally of the treatment chamber 202 in the apparatus 50 of FIG. 2, FIG. 3 shows that one or more flashlamps 256 may alternatively (or in addition) be located externally of a treatment chamber 252. A preferred design is shown in which the composition being treated is conducted through a treatment chamber 252 employing a transmissive treatment conduit (e.g., a quartz tube) 252. The treatment chamber 252 is positioned along one focus of an elliptical reflector 254. A flashlamp 256 is positioned along another focus of the elliptical reflector 254. The flashlamp 256 may optimally be jacketed in a quartz tube for water cooling thereof. Because the light pulses are focused by the elliptical reflector 254 toward the center of the treatment chamber 252, compensation is provided for the light absorption of the composition being treated, so that all of the composition is subjected to uniform light treatment. In a variation (not shown) of the embodiment shown, multiple elliptical reflectors, each having a flashlamp at one focus and the treatment chamber 252 at the other focus, may be utilized if desired.

While various apparatus useful in practicing the methods of the present invention have been described, it will be appreciated by those of skill in the art that various other apparatus, including combinations of the above-described apparatus, may alternatively be employed for these purposes and thus, are equally contemplated herein. For example, a small pulsed light sterilizer could be employed to treat intravenous solutions containing biologically derived compositions, which could, for example, receive intravenous tubing in an incoming port, pass the solution through the pulsed light chamber and out an exit port, and then through the remaining intravenous tubing and into a patient.

In such treatment apparatus, BPL is employed to illuminate and treat a biologically derived composition to reduce the content of pathogen, for example virus and/or bacteria, by at least a factor of ten. Preferably, the treatment method comprises illuminating the biologically derived composition with pulsed light having an intensity of at least about 0.01 J/cm², preferably about 0.02 J/cm² to about 50 J/cm², and most preferably about 0.05 J/cm² to 1.0 J/cm², wherein the energy intensity is measured at the surface of the composition being illuminated. The light pulses are preferably of very short duration. Pulse durations of less than about 100 ms are preferred; durations of between about 10 ns and 100 ms, e.g. about 0.3 ms, are most preferred. In addition to the pulsed light being of high-intensity and short duration, it should be characterized as incoherent, polychromatic light in a broad spectrum. Preferably, the light includes wavelengths from about 170 nm to about 2600 nm (i.e., frequencies of about 1.8x10¹⁵ Hz to about 1.2x10¹⁴ Hz). Because the time between pulses may be very short, e.g., less than about 100 ms, a single, multi-pulse inactivation treatment can be completed in less than a minute for each treatment. If the treatment is of a flowing product being pumped or otherwise circulated (as opposed to a batch treatment), it may be desirable to provide a plurality of flashlamps spaced along the flow path to permit a faster rate of flow. This type of treatment is in sharp contrast to heretofore known methods of reducing the content of pathogens in biologically derived compositions, which can require up to hours to complete.

In order to most efficiently and reliably treat biologically derived compositions in accordance with the methods herein, the composition should be illuminated as fully as possible by the broad-spectrum pulsed light. Thus, it is important that the composition to be treated be contained, at least during treatment, by material that is sufficiently transmissive, for example at least one percent transmissive, to such broad-spectrum pulsed light that inactivation is reliably achieved. Examples of suitable materials include, for example, polyolefins, such as polyethylene and polypropylene, nylon, quartz and sapphire; the '598 patent discusses various polymers suitable for use in pulsed light treatment apparatus.

Different materials may be preferred for forming the containing material depending upon the particular configuration of the pulsed light treatment apparatus in use. For example, where the treatment apparatus employs closely spaced thin plates through which the biologically derived composition passes, such plates are preferably formed of a relatively hard, transmissive material, such as quartz or sapphire. In contrast, where the treatment zone is an intravenous tube, a pliable polymer material will be preferred.

In addition to considering which pulsed light treatment apparatus to use and within which material to contain a biologically derived composition to be treated, certain characteristics of the composition itself should be considered. For example, the transmissivity of the biologically derived composition is important to insuring complete illumination thereof with the broad-spectrum pulsed light. Various components of a biologically derived composition may contribute to reduced transmissivity of the composition to broad-spectrum pulsed light. For example, the presence of whole cells, high concentrations of proteins and/or large quantities of lipids or other macromolecules can all interfere with transmission of broad-spectrum pulsed light through and/or within a biologically derived composition.

Fortunately, the transmissivity of a biologically derived composition can generally be adjusted without permanent damage to the composition by simple dilution which is often commonly practiced in the processing of such compositions so that appropriate techniques for such are well known. If it is desired to improve the transmissivity of blood plasma, for example, to BPL in order to optimize treatment thereof in accordance with the methods herein, the plasma may first be diluted, for example with an appropriate saline solution, then treated with BPL and, following treatment, reconcentrated or further processed to provide the final desired product. Because whole cells present in a biologically derived composition may be irreparably damaged by treatment with pulsed light in accordance with the present methods, these methods are best suited for treating biologically derived compositions that either do not include whole cells or that do not require recovery of intact whole cells following treatment thereof with BPL.

As stated previously, these methods can be advantageously employed to inactivate pathogens present in a biologically derived composition generally without destroying the therapeutic, pharmaceutical, nutritional and/or other desirable properties of the biomolecule(s) of interest within the composition. While the effectivity of some biomolecules of interest may decrease slightly as a result of treatment with broad-spectrum pulsed light, in many such instances the remaining activity will still be acceptable. Furthermore, the effectivity of some biomolecules of interest may in some instances be enhanced by treatment with BPL, which can result in improved or even new therapies.

FIG. 4 is a flow chart wherein some of the various embodiments of the methods of the present invention are illustrated. An undiluted sample 300 of a biologically derived composition to be treated may be diluted to a dilute composition 302 or may be treated without dilution. Three preferred exemplary methods of introducing the biologically derived composition into the treatment zone of the pulsed light treatment apparatus are presented: a tubular flow method 304, a batch treatment method 306 and a plate flow treatment 308. Specific embodiments of the tubular flow 304 method have been described with respect to FIGS. 2 and 3. According to this first alternative, the composition is introduced into the treatment zone of the treatment chamber by a tube or similarly elongated apparatus. This includes for example, treatment in an elliptical treatment apparatus, treatment through an intravenous tube and related methods. Preferably the tube, at least at the point where the composition is illuminated by pulsed light, has a small enough diameter that the entirety of the composition is illuminated.

A second alternative for introducing the biologically derived composition into the treatment zone of the treatment chamber is to treat the composition in batch form 306. In this alternative the composition may, for example, be divided into relatively small quantities and placed within one or more appropriately transmissive containers and illuminated with the pulsed light. The batch method of treatment is particularly useful for pulsed light treatment of media components, such as fetal bovine serum, which can be very quickly and effectively treated in small quantities using the batch method. A third option presented in FIG. 4 is the plate flow 308 apparatus. As described in detail above, the plate flow 308 apparatus comprises two closely spaced, thin plates through which the composition to be treated is caused to flow. Advantageously, this particular option permits treatment of a significant volume of composition at one time by distributing the composition into a thin, wide layer. Alternative means for introducing the biologically derived composition into the treatment zone of the pulsed light treatment chamber will be apparent to those of skill in the art.

Following are some examples of inactivation of specific pathogens employing the methods described herein. In particular, illustrated are the inactivation of HIV-1 and BVDV present in biologically derived compositions. The results of these experiments show that the content of each of the four viruses tested was significantly reduced after treatment with just three high-intensity, short duration pulses of incoherent polychromatic light in a broad spectrum. Significantly, a reduction in viral content greater than three (3) logs was demonstrated for both HIV-1 and CPV after illumination with just a single pulse of broad-spectrum light. A fifth example which follows illustrates inactivation of a bacteria, *E*.*coli*, present in fetal bovine blood serum.

### EXAMPLE 1: INACTIVATION OF SV40

African Green Monkey Kidney (AGMK) cells are inoculated with Simian Virus 40(SV40, ATCC VR-305). The virus is harvested when 75% to 100% of the cells exhibit cytopathic effects (CPE). Stocks of virus are frozen and stored at -60°C in a medium containing 10% to 15% Fetal Bovine Serum (FBS).

AGMK cells are used for titration of the samples containing SV40. The medium used for growth and maintenance of AGMK cells is Eagles Minimum Essential Medium(E-MEM) with 5% - 10% heat-inactivated FBS, 10 mg/mL gentamicin, 100 units/mL penicillin, and 2.5 mg/mL fungizone.

Cell cultures are incubated at 36-38°C in a humidified atmosphere of 5-7% CO₂

A 0.2 mL volume of SV40 in E-MEM with 10% heat-inactivated FBS is added to sterile polyethylene sample containers. Twelve replicate samples are made. Nine of the samples are treated with BPL. Of these, three samples are treated with a single light pulse, three samples are treated with two pulses, and three samples are treated with three pulses. Three untreated samples serve as controls.

After exposure, a titration is performed by preparing ten-fold dilutions from each viral sample in a 96-well microculture plate seeded with AGMK. SV40 is allowed to adsorb for 1 - 2 hours, then removed from the wells and replaced with fresh culture medium. A titration of RPMI (without phenol red) is performed as a control. All titrations are plated in quadruplicate, monitored for up to 10 days for the presence of CPE and/or cytotoxicity. Results are set forth in Table 1.

**Table 1:**

| Treatment of SV40 with BPL. | | | | |
|---|---|---|---|---|
| Replicate # | 0 Pulses | 1 Pulse | 2 Pulses | 3 Pulses |
| 1 | | 5.62x10³ | 3.16x10³ | 1.79x10³ |
| 2 | 1.78x10⁵ | 3.16x10³ | 3.16x10³ | 1.78x10² |
| 3 | | 5.62x10³ | 1.78x10³ | 3.16x10² |
| Average | 1.78x10⁵ | 4.8x10³ | 2.7x10³ | 7.61x10² |

Post-exposure titrations are compared to the pre-exposure titration, in order to determine if viral infectivity was reduced by exposure to BPL. Example 1 shows a 2-3 log reduction of SV40. The highest available titre is used in this example. Greater reduction of viruses is expected when higher starting titres are used, more pulses are applied, or the medium is diluted to a lower concentration of protein.

### EXAMPLE 2: INACTIVATION OF HIV-1

CCRF-CEM cells are inoculated with HIV Type I strain HTLVIIIB (Vanderbilt University, Nashville, TN). HIV-1 is harvested when infectivity reaches a minimum of 80% as determined by an immunofluorescence assay (IFA). Stocks of virus are frozen and stored at -60°C in a medium containing 10% to 15% FBS.

MT-2 cells (NIH AIDS Research and Reference Reagent Program, catalog number 237) are used for titration of the samples containing HIV-1. The medium used for growth and maintenance of MT-2 cells is RPMI 1640 (with phenol red) supplemented with 2 mM L-glutamine, 25 mM Hepes, 2 g/L NaHCO₃, 15% heat-inactivated FBS and 50 mg/ml gentamicin. Cell cultures are incubated at 36-38°C in a humidified atmosphere of 5-7% CO₂.

A 0.2 mL volume of HIV in RPMI with 15% heat inactivated FBS is added to sterile polyethylene sample containers. Twelve replicate samples are made. Nine of the samples are treated with BPL. Of these, three samples are treated with a single light pulse, three samples are treated with two pulses, and three samples are treated with three pulses. Three untreated samples serve as controls.

After exposure, a titration is performed by preparing ten-fold dilutions from each viral sample in a 96-well microculture plate seeded with MT-2 cells. HIV-1 dilutions remain in the wells throughout the titration period. A titration of RPMI (without phenol red) is performed as a control. All titrations are plated in quadruplicate, monitored for up to 10 days for the presence of CPE and/or cytotoxicity. Results are set forth in Table 2.

**Table 2:**

| Treatment of HIV-1 with BPL. | | | | |
|---|---|---|---|---|
| Replicate # | 0 pulses | 1 pulse | 2 pulses | 3 pulses |
| #1 | | 1x10³ | 5.62x10¹ | No survivors* |
| #2 | 1x10⁶ | 3.16x10³ | No survivors* | No survivors* |
| #3 | | 3.16x10³ | No survivors* | No survivors* |
| AVERAGE | 1x10⁶ | 2.44x10³ | No survivors* | No survivors* |

| | | | | |
|---|---|---|---|---|
| *The detection limit of this test was ≤3.16x10¹. | | | | |

Post-exposure titrations are compared to the pre-exposure titration, in order to determine if viral infectivity was reduced by exposure to BPL. Example 2 shows that 5 to 6 log reduction of HIV-1 occurred using BPL.

The highest available titre is used in this example. Greater reduction of viruses is expected when higher starting titres are used, more pulses are applied, or the medium is diluted to a lower concentration of protein.

### EXAMPLE 3: INACTIVATION OF CPV

A-72 cells (ATCC CRL 1542) are inoculated with CPV (ATCCVR-2017). The virus is harvested when 75% to 100% of the cells exhibit cytopathic effects (CPE). Stocks of virus are frozen and stored at -60°C in a medium containing 10% to 15% FBS.

A-72 cells are used for titration of the samples containing CPV. The medium used for growth and maintenance of A-72 cells is E-MEM supplemented with 5% - 10%heat-inactivated FBS, 10 mg/mL gentamicin, 100 units/mL penicillin, and 2.5 mg/mL fungizone. Cell cultures are incubated at 36-38°C in a humidified atmosphere of 5-7% CO₂.

A 0.2 mL volume of CPV in E-MEM with 5% heat inactivated FBS is added to sterile polyethylene sample containers. Twelve replicate samples are made. Nine of the samples are treated with BPL. Of these, three samples are treated with a single light pulse, three samples are treated with two pulses, and three samples are treated with three pulses. Three untreated samples serve as controls.

After exposure, a titration is performed by preparing ten-fold dilutions from each viral sample in a 96-well microculture plate seeded with A-72. CPV is allowed to adsorb for 1 - 2 hours, then removed from the wells and replaced with fresh culture medium.
Supernatant samples are transferred from the CPV titration plate to fresh plates for testing by hemagglutination (HA). A titration of RPMI(without phenol red) is performed as a control. All titrations are plated in quadruplicate, monitored for up to 10 days for the presence of CPE and/or cytotoxicity. Results are set forth in Table 3.

**Table 3:**

| Treatment of CPV with BPL. | | | | |
|---|---|---|---|---|
| Replicate # | 0 pulses | 1 pulse | 2 pulses | 3 pulses |
| #1 | | 3.16x10² | No survivors* | 3.16x10² |
| #2 | 1x10⁶ | 3.16x10² | No survivors* | No survivors* |
| #3 | | 3.16x10² | No survivors* | No survivors* |
| AVERAGE | 1X10⁶ | 3.16x10² | No survivors* | 3.16x10² |

| | | | | |
|---|---|---|---|---|
| *The detection limit of this test was ≤3.16x10¹. | | | | |

Post-exposure titrations are compared to the pre-exposure titration, in order to determine if viral infectivity was reduced by exposure to BPL. Example 3 shows that 3 to 4 log reduction of CPV occurred using BPL.

The highest available titre is used in this example. Greater reduction of viruses is expected when higher starting titres are used, more pulses are applied, or the medium is diluted to a lower concentration of protein.

### EXAMPLE 4: INACTIVATION OF BVDV

Bovine Turbinate (BT) cells (ATCC CRL 1390) are inoculated with BVDV (ATCC VR-534). The virus is harvested when 75% to 100% of the cells exhibit cytopathic effects (CPE). Stocks of virus are frozen and stored at -60°C in a medium containing 10% to 15% FBS.

BT cells are used for titration of the samples containing BVDV. The medium used for growth and maintenance of BT cells is E-MEM supplemented with 15% horse serum.

Cell cultures are incubated at 36-38°C in a humidified atmosphere of 5-7% CO₂.

A 0.2 mL volume of BVDV in E-MEM with 5% heat inactivated FBS is added to sterile polyethylene sample containers. Twelve replicate samples are made. Nine of the samples are treated with BPL. Of these, three samples are treated with a single light pulse, three samples are treated with two pulses, and three samples are treated with three pulses. Three untreated samples serve as controls.

After exposure, a titration is performed by preparing ten-fold dilutions from each viral sample in a 96-well microculture plate seeded with A-72. BVDV is allowed to adsorb for 1 - 2 hours, then removed from the wells and replaced with fresh culture medium. A titration of RPMI (without phenol red) is performed as a control. All titrations are plated in quadruplicate, monitored for up to 10 days for the presence of CPE and/or cytotoxicity. Results are set forth in Table 4.

**Table 4:**

| Treatment of BVDV with BPL. | | | | |
|---|---|---|---|---|
| | 0 pulses | 1 pulse | 2 pulses | 3 pulses |
| Replicate 1 | | 1x10⁴ | 1.78x10³ | 1x10² |
| Replicate 2 | 3.16x10⁶ | 3.16x10³ | 5.62x10² | 1x10² |
| Replicate 3 | | 1x10⁴ | 5.62x10² | 1.78x10² |
| AVERAGE | 3.16x10⁶ | 7.72x10³ | 9.68x10² | 1.26x10² |

Post-exposure titrations are compared to the pre-exposure titration in order to determine if viral infectivity was reduced by exposure to BPL. Example 4 shows that 4 log reduction of BVDV occurred using BPL.

The highest available titre is used in this example. Greater reduction of viruses is expected when higher starting titres are used, more pulses are applied, or the medium is diluted to a lower concentration of protein.

### EXAMPLE 5: INACTIVATION OF E. COLI

A sample of 15% FBS was inoculated with *E*. *coli* (ATCC26) and permitted to grow overnight, at 32°C, to a final concentration of 8.8x10⁴ CFU/mL (4.9 log CPU/mL). 200 µL of the contaminated serum (containing 17,600 CFU, *i*.*e*., 4.25 log CFU) was then illuminated with 3 short-duration pulses of broad-spectrum light (1.0 J/cm²/flash) in accordance with the methods described herein. Serial dilutions (10x) of the sample were made (using 0.05M phosphate buffer) and plated onto standard agar plates. The plates were incubated at 32°C and counted at 24 and 48 hours.

After 48 hours of incubation, no surviving *E. coli* was observed on any plates. Given a sensitivity level of 20 CFU (*i*.*e*., 1.3 log CFU), this test demonstrated that treatment of *E*. *coli*-innoculated FBS, with just 3 pulses of broad-spectrum light resulted in about a 3 log reduction in bacterial content. (4.25 log CFU - 1.3 log CFU = 2.95 log CFU.)

While the invention herein disclosed has been described by means of specific embodiments and applications thereof, numerous modifications and variations can be made thereto, by those of skill in the art, without departing from the scope of the invention as set forth in the following claims.

## Claims

1. A method for reducing the content of a pathogen present in a biologically derivable composition comprising illuminating the composition with at least one high-intensity, short duration pulse of incoherent polychromatic light in a broad spectrum wherein the light intensity is at least 0.01 J/cm², the pulse duration is less than 100 ms and the light wavelengths are between 170 nm to 2600 nm, such that the content of pathogen is reduced by a factor of at least 10,
wherein the biologically, derivable composition is a blood plasma composition, a Factor VIII composition, a Factor IX composition, an antithrombin composition, a transferrin composition, an albumin composition, an immunoglobulin composition, a monoclonal antibody composition, a viral vector composition, a heparin composition, a collagen composition, an insulin composition or a blood serum albumin composition.

2. A method according to Claim 1, wherein the pathogen is a virus, bacteria, pyrogen, toxin, fungi or a prion.

3. A method according to Claim 2, wherein the pathogen is a virus selected from human immunodeficiency virus (HIV)-1, HIV-2, hepatitis A, hepatitis B, hepatitis C, HTLV-I, HTLV-II, cytomegalovirus, simian vacuolating virus 40, bovine viral diarrhea virus and canine parvovirus.

4. A method according to any preceding Claim, wherein said illuminating step comprises illuminating the biologically derived composition with at least two high-intensity, short duration pulses of incoherent polychromatic light in a broad spectrum.

5. A method according to any preceding Claim, wherein said biologically derivable composition comprises a biomolecule of interest which is not destroyed by the illuminating step.

6. A method according to any preceding Claim, further comprising the steps of:
diluting the biologically derived composition prior to said illuminating step; and
concentrating the biologically derived composition after said illuminating step.

7. A method according to any preceding Claim, further comprising the steps of:
providing a broad-spectrum pulsed light apparatus comprising a treatment zone including at least one flashlamp and two opposing transmissive plates; and
causing the biologically derived composition to flow between the two opposing transmissive plates;
wherein
said illuminating of said biologically derived composition is performed while it is located between the two opposing transmissive plates.

8. A method according to Claim 1, wherein the biologically derivable composition is bovine serum, sheep blood, bovine insulin, bovine transferrin, bovine heparin, collagen, amino acids, peptones, peptides, or monoclonal antibodies or proteins from a genetically engineered mammalian cell line.

9. A method according to Claim 8, wherein the pathogen is a virus and the biologically derived composition is bovine serum, sheep blood, bovine insulin, bovine transferrin, bovine heparin, collagen, amino acids, peptones, peptides or monoclonal antibodies.

10. A method according to Claim 8 or 9, wherein said illuminating step results in at least a two log reduction in active pathogen content.

## Patentansprüche

1. Verfahren zur Verringerung des Gehalts eines Pathogens, das in einer auf biologischem Wege erhältlichen Zusammensetzung vorhanden ist, das die Bestrahlung der Zusammensetzung mit mindestens einem inkohärenten polychromatischen Lichtpuls hoher Intensität und kurzer Dauer mit breitem Spektrum umfasst, worin die Lichtintensität mindestens 0,01 J/cm² und die Pulsdauer weniger als 100 ms beträgt und die Lichtwellenlängen zwischen 170 und 2.600 nm liegen, in einer solchen Weise, dass das Pathogen um mindestens einen Faktor 10 verringert wird, worin die auf biologischem Wege erhältliche Zusammensetzung eine Blutplasmazusammensetzung, eine Faktor VIII-Zusammensetzung, eine Faktor IX-Zusammensetzung, eine Antithrombinzusammensetzung, eine Transferrinzusammensetzung, eine Albuminzusammensetzung, eine Immunoglobulinzusammensetzung, eine monoklonale Antikörperzusammensetzung, eine Viralvektorzusammensetzung, eine Heparinzusammensetzung, eine Kollagenzusammensetzung, eine Insulinzusammensetzung oder eine Blutserumalbuminzusammensetzung ist.

2. Verfahren gemäss Anspruch 1, worin das Pathogen ein Virus, ein Bakterium, ein Pyrogen, ein Toxin, ein Pilz oder ein Prion ist.

3. Verfahren gemäss Anspruch 2, worin das Pathogen ein Virus ist, ausgewählt aus humanem Immunschwächevirus (HIV)-1, HIV-2, Hepatitis A, Hepatitis B, Hepatitis C, HTLV-I, HTLV-II, Cytomegalovirus, simianvacuolierendem Virus 40, Rinderviraldiarrhoevirus und Hundeparvovirus.

4. Verfahren gemäss mindestens einem der vorhergehenden Ansprüche, worin der Bestrahlungsschritt die Bestrahlung der auf biologischem Wege erhaltenen Zusammensetzung mit mindestens zwei inkohärenten polychromatischen Lichtpulsen hoher Intensität und kurzer Dauer mit einem breiten Spektrum umfasst.

5. Verfahren gemäss einem der vorhergehenden Ansprüche, worin die auf biologischem Wege erhältliche Zusammensetzung ein Biomolekül von Interesse umfasst, das durch den Bestrahlungsschritt nicht zerstört wird.

6. Verfahren gemäss mindestens einem der vorhergehenden Ansprüche, das ferner folgende Schritte umfasst:
Verdünnung der auf biologischem Wege erhaltenen Zusammensetzung vor dem Bestrahlungsschritt; und
Einengung der auf biologischem Wege erhaltenen Zusammensetzung nach dem Bestrahlungsschritt.

7. Verfahren gemäss mindestens einem der vorhergehenden Ansprüche, das ferner folgende Schritte umfasst:
Bereitstellung einer gepulsten Breitbandbeleuchtungsvorrichtung, die eine Behandlungszone, die mindestens eine Blitzlampe und zwei einander gegenüberliegende durchlässige Platten einschliesst, umfasst; und
Bewirken des Flusses der auf biologischem Wege erhaltenen Zusammensetzung zwischen den einander gegenüberliegenden durchlässigen Platten; worin
die Bestrahlung der auf biologischem Wege erhaltenen Zusammensetzung durchgeführt wird, während sie zwischen den einander gegenüberliegenden durchlässigen Platten befindlich ist.

8. Verfahren gemäss Anspruch 1, worin die auf biologischem Wege erhältliche Zusammensetzung Rinderserum, Schafsblut, Rinderinsulin, Rindertransferrin, Rinderheparin, Kollagen, Aminosäuren, Peptone, Peptide oder monoklonale Antikörper oder Proteine aus einer genmanipulierten Säugerzellinie darstellt.

9. Verfahren gemäss Anspruch 8, worin das Pathogen ein Virus ist, und die auf biologischem Wege erhaltene Zusammensetzung ist Rinderserum, Schafsblut, Rinderinsulin, Rindertransferrin, Rinderheparin, Kollagen, Aminosäuren, Peptone, Peptide oder monoklonale Antikörper.

10. Verfahren gemäss Anspruch 8 oder 9, worin der Bestrahlungsschritt zu einer mindestens zwei logfachen Reduktion des aktiven Pathogengehalts führt.

## Revendications

1. Procédé pour réduire la quantité d'un agent pathogène présent dans une composition d'origine biologique, comprenant l'éclairement de la composition avec au moins une impulsion de courte durée, à forte intensité, de lumière polychromatique incohérente dans un large spectre, dans lequel l'intensité lumineuse est d'au moins 0,01 J/cm², la durée d'impulsion est inférieure à 100 ms et les longueurs d'ondes de la lumière sont comprises entre 170 nm et 2600 nm, de telle sorte que la quantité d'agent pathogène soit réduite d'un facteur d'au moins 10,
la composition d'origine biologique étant une composition de plasma sanguin, une composition de Facteur VIII, une composition de Facteur IX, une composition d'antithrombine, une composition de transferrine, une composition d'albumine, une composition d'immunoglobuline, une composition d'anticorps monoclonal, une composition de vecteur viral, une composition d'héparine, une composition de collagène, une composition d'insuline ou une composition de sérumalbumine sanguin.

2. Procédé suivant la revendication 1, dans lequel l'agent pathogène est un virus, une bactérie, un agent pyrogène, une toxine, un champignon ou un prion.

3. Procédé suivant la revendication 2, dans lequel l'agent pathogène est un virus choisi entre le virus d'immunodéficience humaine (HIV)-1, le HIV-2, le virus de l'hépatite A, le virus de l'hépatite B, le virus de l'hépatite C, le HTLV-I, le HTLV-II, le cytomégalovirus, le virus vacuolisant simien 40, le virus de la diarrhée virale bovine et le parvovirus canin.

4. Procédé suivant l'une quelconque des revendications précédentes, dans lequel ladite étape d'éclairement comprend l'éclairement de la composition d'origine biologique avec au moins deux impulsions de courte durée, à forte intensité, de lumière polychromatique incohérente dans un large spectre.

5. Procédé suivant l'une quelconque des revendications précédentes, dans lequel ladite composition d'origine biologique comprend une biomolécule intéressante qui n'est pas détruite par l'étape d'éclairement.

6. Procédé suivant l'une quelconque des revendications précédentes, comprenant en outre les étapes consistant :
à diluer la composition d'origine biologique avant ladite étape d'éclairement ; et
à concentrer la composition d'origine biologique après ladite étape d'éclairement.

7. Procédé suivant l'une quelconque des revendications précédentes, comprenant en outre les étapes consistant :
à fournir un appareil à lumière pulsée et à large spectre, comprenant une zone de traitement comportant au moins une lampe éclair et deux plaques de transmissivité opposées ; et
à faire circuler la composition d'origine biologique entre les deux plaques de transmissivité opposées ; dans lequel
ledit éclairement de ladite composition d'origine biologique est effectué tandis qu'elle est située entre les deux plaques de transmissivité opposées.

8. Procédé suivant la revendication 1, dans lequel la composition d'origine biologique est le sérum bovin, le sang de mouton, l'insuline bovine, la transferrine bovine, l'héparine bovine, le collagène, des aminoacides, des peptones, des peptides ou des anticorps monoclonaux ou protéines provenant d'une lignée de cellules de mammifère modifiées génétiquement.

9. Procédé suivant la revendication 8, dans lequel l'agent pathogène est un virus et la composition d'origine biologique est le sérum bovin, le sang de mouton, l'insuline bovine, la transferrine bovine, l'héparine bovine, le collagène, des aminoacides, des peptones, des peptides ou des anticorps monoclonaux.

10. Procédé suivant la revendication 8 ou 9, dans lequel ladite étape d'éclairement a pour résultat une réduction logarithmique d'au moins un facteur deux de la quantité d'agent pathogène actif.
